# EUROPEAN PATENT APPLICATION

(11) **EP 3 763 281 A1**
(43) Date of publication of application: **13.01.2021**
(21) Application number: 19763780.4
(22) Date of filing: 04.03.2019
(51) Int. Cl.: A61B 3/10

(54) **OCULAR FUNDUS IMAGE PROCESSING DEVICE AND OCULAR FUNDUS IMAGE PROCESSING PROGRAM**

(30) Priority: 05.03.2018 JP 2018039164; 18.04.2018 JP 2018080131
(71) Applicant: Nidek Co., Ltd., Gamagori-shi, Aichi, 443-0038 (JP)
(72) Inventor: KUMAGAI, Yoshiki, Gamagori-shi Aichi 443-0038 (JP); HONDA, Naoto, Gamagori-shi Aichi 443-0038 (JP)
(74) Representative: Hoefer & Partner Patentanwälte mbB
(86) International application number: PCT/JP2019/008478
(87) International publication number: WO 2019/172206

(57) **Abstract**

An ocular fundus image processing device processes an ocular fundus image of a subject's eye. The ocular fundus image processing device includes an image acquisition means for acquiring the ocular fundus image imaged by imaging means, and an analysis means for analyzing the ocular fundus image. The imaging means generates the ocular fundus image on the basis of a received signal per wavelength obtained by scanning measurement light that includes a plurality of wavelengths by scanning means and receiving the measurement light reflected from the ocular fundus of the subject's eye with a light receiving element. The analysis means detects findings in the ocular fundus image generated on the basis of the received signal per wavelength. This facilitates image diagnosis of multicolor images imaged by a scanning multicolor imaging device.

## Description

### Technical Field

The present invention relates to a fundus image processing device for processing a fundus image of a subject eye, and a fundus image processing program.

### Background Art

As an ophthalmologic imaging device in the related art, for example, an optical coherence tomography (OCT), a fundus camera, a scanning laser ophthalmoscope (SLO), and the like are known.

### Citation List

### Patent Literature

Patent Literature 1: JP-A-2015-104581

### Summary of Invention

Incidentally, there is a scanning multi-color imaging device that acquires a multi-color image by scanning with a measurement light including a plurality of wavelengths for imaging a subject eye. For example, since the scanning multi-color imaging devices such as a multi-color SLO (MCSLO) can catch a minute change in the fundus more than from a fundus image in the related art, it is expected that the more accurate diagnosis can be performed. However, because the multi-color image captured by the scanning multi-color imaging device is different from the fundus image in the related art in view of picture, it is necessary to learn new knowledge for the image interpretation.

A technical object of the present disclosure is to provide a fundus image processing device and a fundus image processing program that can easily perform an image diagnosis on a multi-color image captured by a scanning multi-color imaging device.

In order to solve the above problems, the present disclosure includes following configurations.
(1) A fundus image processing device that processes a fundus image of a subject eye, including:
   image acquisition means for acquiring the fundus image captured by imaging means; and
   analysis means for analyzing the fundus image,
   in which the imaging means performs scanning with measurement light including a plurality of wavelengths by scanning means, and generates the fundus image based on a received light signal for each wavelength obtained from a light receiving element which receives the measurement light reflected from a fundus of the subject eye, and
   the analysis means detects findings for the fundus image generated based on the received light signal for each wavelength.
(2) A fundus image processing program executed in a fundus image processing device that processes a fundus image of a subject eye, and executed by a processor of the fundus image processing device to cause the fundus image processing device to perform:
   a generation step of performing scanning with measurement light including a plurality of wavelengths by scanning means, and generating the fundus image based on a received light signal for each wavelength obtained from a light receiving element which receives the measurement light reflected from a fundus of the subject eye;
   an image acquisition step of acquiring the fundus image generated in the generation step; and
   an analysis step of detecting findings for the fundus image generated based on the received light signal for each wavelength.

### Brief Description of Drawings

[FIG. 1] FIG. 1 is a schematic configuration diagram illustrating a configuration of an ophthalmologic imaging device according to a present example.
[FIG. 2] FIG. 2 is a diagram illustrating an optical configuration of an MCSLO.
[FIG. 3] FIG. 3 is a diagram illustrating a flowchart of a control operation.
[FIG. 4A] FIG. 4A is a diagram illustrating an example of an MCSLO image.
[FIG. 4B] FIG. 4B is a diagram illustrating an example of an MCSLO image.
[FIG. 5] FIG. 5 is a diagram for explaining learning of a mathematical model.
[FIG. 6] FIG. 6 is a diagram illustrating a findings map.
[FIG. 7] FIG. 7 is a diagram illustrating an example of an output result of findings.
[FIG. 8A] FIG. 8A is a diagram illustrating an example of a fundus image captured by a different imaging device.
[FIG. 8B] FIG. 8B is a diagram illustrating an example of a fundus image captured by a different imaging device.
[FIG. 9] FIG. 9 is a diagram for explaining normalization of data input to the mathematical model.
[FIG. 10] FIG. 10 is a diagram illustrating how each of the channels of a fundus image are weighted.
[FIG. 11] FIG. 11 is a diagram illustrating an example of an output result of findings.

### Description of Embodiments

### <Embodiment>

Hereinafter, an embodiment of a fundus image processing device according to the present disclosure will be described. The fundus image processing device (for example, a fundus image processing device 100) in the present embodiment performs processing on a fundus image of a subject eye. The fundus image processing device includes, for example, an image acquisition unit (for example, an image acquisition unit 1) and an analysis unit (for example, an analysis unit 2).

The image acquisition unit acquires, for example, a multi-color fundus image of the subject eye captured by an imaging unit (for example, an MCSLO 200). The image acquisition unit is connected to, for example, the imaging unit that images the multi-color fundus image or a storage unit that stores the multi-color fundus image by wired (USB cable, LAN cable, IEEE1394 cable, or the like) or wireless communication means. The image acquisition unit acquires the multi-color fundus image from the imaging unit or the storage unit, and the like via the communication means. The image acquisition unit acquires, for example, the multi-color fundus image such as a plurality of fundus image divided for each wavelength, or a multi-channel image in which two or more of the fundus images are combined.

The imaging unit is, for example, a scanning multi-color imaging device. The imaging unit includes, for example, a light source, a scanning unit, and a light receiving element. The light source emits measurement light including a plurality of wavelengths, for example. The scanning unit scans and irradiates the subject eye with the measurement light, for example. The scanning unit may be configured to perform the scanning with the measurement light by a galvano mirror, or may be configured to perform the scanning with the measurement light by moving an aperture. The light receiving element receives, for example, reflected light (return light) of the measurement light reflected from the fundus of the subject eye.

The imaging unit acquires a received light signal for each wavelength. For example, the imaging unit may irradiate the subject eye with the measurement light at different timings for each wavelength, and may acquire the received light signal for each wavelength obtained from the light receiving element which receives the return light. In addition, the imaging unit may split the return light when the subject eye is irradiated with the measurement light (for example, white light) including the plurality of wavelengths for each wavelength by a light separation unit such as a dichroic mirror, and then, may receive the result with the light receiving element. For example, the imaging unit may generate a single-channel fundus image based on the received light signal for each wavelength, or may acquire a multi-channel image in which two or more of the fundus images for each wavelength are combined.

The analysis unit detects findings for the multi-color fundus image acquired by the image acquisition unit. The findings are features relating to diseases such as retinal hemorrhage, capillary aneurysm, macular anterior membrane, vitiligo, edema, retinal detachment, and laser scar. For example, the analysis unit detects the presence or absence of the findings, or the position thereof, by performing image processing on the multi-color fundus image.

As described above, the fundus image processing device in the present embodiment can provide the user with information useful for diagnosis by detecting the findings in the multi-color fundus image acquired by the image acquisition unit.

The measurement light may include visible light in the wavelength range such as red, green or blue. By imaging the fundus with the measurement light having the red, green or blue wavelength ranges, it is possible to obtain a good image of layers of different depths such as a retinal surface layer, a vascular layer, and a retinal deep layer centered on the retinal pigment epithelium (RPE). Of course, other wavelengths may be included. For example, the measurement light may include light rays having wavelength ranges such as ultraviolet, violet, blue green, yellow green, yellow, orange, or infrared.

The analysis unit may detect the findings for each of the plurality of fundus images generated for each wavelength. In addition, the analysis unit may detect the findings for the multi-channel image in which equal to or more than two channels are combined in the plurality of fundus images generated for each wavelength. Of course, findings detection may be performed for all fundus images and multi-channel images for each wavelength. As described above, the more information, or the diversified information can be obtained by detecting the findings for various forms of multi-color fundus image.

The analysis unit may analyze the fundus image with using a mathematical model trained by a machine learning algorithm. For example, a neural network, a random forest, a boosting, a support vector machine (SVM), and the like are generally known as the machine learning algorithms.

The neural network is a mathematical model in which a plurality of units that output results of performing some processing on an input signals are connected to each other. A convolutional network, which is a kind of neural network, is an effective method for the image recognition problem. Typical image recognition problems include object identification, object detection, and segmentation, and various models are proposed for each problem. The object identification is a problem of identifying what is captured in the image, and models such as AlexNet, GoogLeNet, and the like are proposed. The object detection is a problem that identifies a position and orientation of an object as a rectangle, and models such as YOLO are proposed. The semantic segmentation is a problem of identifying a position of the object at a pixel level, and models such as U-Net are proposed. In addition, other than those described above, a model of a convolutional network is proposed for various problems targeting super-resolution images for increasing the image resolution or the images for colorization that adds the colors to the monochrome images. A recursive neural network is an example of a neural network model other than a convolutional network. The recursive neural network is a model that can handle time series data and is an effective method for natural language processing.

The boosting is a method of generating a strong identifier by combining a plurality of weak identifiers. The strong identifier is constructed by sequentially learning the simple and weak identifiers.

The random forest is a method in which a large number of decision trees are generated by performing the learning based on training data that is randomly sampled. When the random forest is used, a plurality of decision trees that are learned as the identifier are branched, and the results obtained from each decision tree are averaged (or majority voted).

The SVM is a method of forming a two-class pattern identifier using a linear input element. The SVM learns the parameters of the linear input element based on, for example, a criterion (hyperplane separation theorem) of finding a margin-maximized hyperplane that maximizes a distance from each data point from the training data.

The mathematical model refers to, for example, a structure expressing a relationship between the input data and the output data. The mathematical model is optimized based on the training data set. The training data set is a set of input training data and output training data. The input training data is sample data input to the mathematical model. For example, the image of the subject eye captured in the past is used as the input training data. The output training data is sample data of values that the mathematical model should predict from the input training data. For example, a map indicating a region where the findings of the image are present or a label indicating whether or not the findings are present in the image are used for the output training data. The mathematical model is optimized so that the output when a certain input training data is input is close to the corresponding output training data. For example, in the neural network, the weight applied when the output of a unit is input to the next connected unit is updated.

For example, the analysis unit inputs a multi-color fundus image into the mathematical model and outputs the findings. For example, the mathematical model outputs a map indicating the presence or absence of the findings and a region where the findings are present. As described above, the analysis unit automatically performs the detection of the findings with using the trained mathematical model.

The analysis unit may analyze the fundus image with using a different mathematical model for each wavelength (each channel) corresponding to the image. For example, the analysis unit may analyze the fundus image with using a surface layer mathematical model which learned images of a first channel and a deep layer mathematical model which learned images of a second channel different from the first channel. As described above, due to using the different mathematical models for each channel, it is easy to detect even when a plurality of findings overlap. For example, it is easy to detect the multiple findings that have the same two-dimensional position in the vertical and horizontal directions but different only in the depth direction. Of course, the fundus image may be analyzed with using not only the two mathematical models for the retinal surface layer and the retinal deep layer but also three or more mathematical models. For example, the mathematical model for the retinal surface layer, retinal intermediate layer, retinal deep layer, or the like may be used.

For the surface layer mathematical model may learn, for example, a green channel image which is based on a received light signal of green light and a blue channel image which is based on a received light signal of blue light. Since the light having a short wavelength such as the green light and the blue light is mainly reflected from the retinal surface layer, these fundus images have a lot of information on the retinal surface layer. In addition, the deep layer mathematical model may learn a red channel image which is based on a received light signal of red light. Since the light having a long wavelength such as red light is mainly reflected from the retinal deep layer, the red channel image has a lot of information on the retinal deep layer. As described above, the analysis unit may use the mathematical model that learned the image which is based on the received light signal of light having the short wavelength as the surface layer mathematical model, and may use the mathematical model that learned the image which is based on the received light signal of light having the long wavelength as the deep layer learning model.

The imaging unit may be a multi-color SLO (hereinafter, abbreviated as an MCSLO). The MCSLO is a scanning laser ophthalmoscope capable of imaging a multi-color fundus image based on a received light signal for each wavelength by, for example, scanning the subject eye with the measurement light including a plurality of wavelengths.

The analysis unit may output reference information used for the diagnosis of the subject eye based on the findings detected from the fundus image. At this time, a mathematical model that performed the machine learning for outputting the reference information may be used for the diagnosis. For example, the analysis unit may use a diagnostic mathematical model that learned a set of information on the presence or absence of each finding and a result of diagnosis of the subject eye. In this case, the analysis unit may output the reference information used for the diagnosis of the subject eye by inputting the information on the presence or absence of each finding into the diagnostic mathematical model.

In addition, if there is an error in the result of detection of the findings, the user may correct the findings information to be input to the diagnostic mathematical model. In this way, when performing the image diagnosis of the subject eye with using the mathematical model, it becomes possible to confirm the information that is the basis of the diagnosis through the detection of the findings. The findings information used here is considered to be more simply and more strongly indicates the information useful for the diagnosis than the original image from which findings information was detected. Therefore, when performing image diagnosis on the subject eye with using the mathematical model, even when the learning data is small due to a fact that the detection of findings is used, it is highly possible that the learning of the diagnostic model can be appropriately performed. In addition, a good result of detection of the findings can be obtained by using the detection of the findings as the problem of finding a map indicating the area where the findings are present in the fundus image even when the learning data is comparatively small. In this case, it is possible to obtain the information on the presence or absence of the findings in the image and the information on the presence or absence of the findings in each partial area of the image from the map indicating the region where the findings are present, and the information may be input to the diagnostic mathematical model. Of course, the analysis unit may detect the disease from the multi-color fundus image without using the detection of the findings.

A processor of the fundus image processing device may execute the fundus image processing program. The fundus image processing program includes, for example, a generation step, an image acquisition step, and an analysis step. The generation step is a step of generating the fundus image based on the received light signal for each wavelength, which can be obtained by, for example, performing scanning with the measurement light having a plurality of wavelengths by the scanning means, and then, by receiving the measurement light reflected from the fundus of the subject eye by the light receiving element. The image acquisition step is a step of acquiring the fundus image generated in the generation step. The analysis step is a step of detecting the findings in the fundus image generated based on the received light signal for each wavelength. The fundus image processing program may be stored in, for example, the storage unit of the fundus image processing device, or may be stored in an external storage medium.

### <Example>

Hereinafter, an example of the fundus image processing device according to the present disclosure will be described. A fundus image processing device 100 in the present example detects the findings by analyzing the fundus image. The findings are features relating to diseases such as retinal hemorrhage, capillary aneurysm, macular anterior membrane, vitiligo, edema, retinal detachment, and laser scar.

The fundus image processing device 100 includes, for example, an image acquisition unit 1, an analysis unit 2, a storage unit 4, an operation unit 5, a display control unit 6 or a display unit 7. The image acquisition unit 1 acquires a multi-color SLO image of the subject eye. The multi-color SLO image is, for example, a 3-channel image of RGB (red, green, blue) acquired by a multi-color SLO (abbreviated as MCSLO) 200. The image acquisition unit 1 is connected to the MCSLO 200 via wired or wireless communication means. For example, the image acquisition unit 1 receives the MCSLO image from the MCSLO 200 via the communication means and stores the image in the storage unit 4 or the like. The image acquisition unit 1 may acquire the MCSLO image from an external storage device such as an HDD or a USB memory connected via the communication means.

The analysis unit 2 detects findings by analyzing the acquired MCSLO image. For example, the analysis unit 2 performs image analysis with using the mathematical model trained by a machine learning algorithm. For example, the mathematical model is trained to output the presence or absence of each finding, the position thereof, and the like when the MCSLO image is input. The analysis unit 2 outputs a result of detection of findings by inputting the MCSLO image to the mathematical model. The result of detection of the findings by the analysis unit 2 is sent to the display unit 7, the storage unit 4, or the like.

The storage unit 4 stores various programs relating to the control of the fundus image processing device 100, various image data, result of analysis, and the like. The display control unit 6 controls the display of the display unit 7. The display unit 7 displays the image acquired by the image acquisition unit 1, the result of analysis by the analysis unit 2, and the like. The display unit 7 may be a touch panel type display. In this case, the display unit 7 is also used as an operation unit.

The image acquisition unit 1, the analysis unit 2, the storage unit 4, and the display control unit 6 may be realized by a processor (for example, a CPU) of a computer used as the image processing device 100 executing various programs, or may be provided as independent control boards.

The fundus image processing device 100 may be, for example, a personal computer. For example, a desktop PC, a notebook PC, or a tablet PC may be used as the fundus image processing device 100. Of course, a server may be used as the device. In addition, the fundus image processing device 100 may be a computer stored inside of an ophthalmologic imaging device or the like.

### <Analysis Processing using Mathematical Model>

Next, the image analysis processing performed by the analysis unit 2 will be described. The analysis unit 2 performs the analysis with using a mathematical model trained by a machine learning algorithm. The machine learning algorithm is, for example, a neural network, a random forest, a boosting, a support vector machine (SVM), or the like.

The mathematical model described above can perform prediction on unknown data by optimizing the data with using appropriate training data set and algorithm. For example, in a case of the neural network, the model is optimized with using a backpropagation method. Here, the training data set is a set of data in which the input of a problem for the model to learn and the output of the correct answers of the problem are paired. In the optimization, the data is sampled from the training data set, the sampled data is input to the model, and the values in the model are updated so that a difference between the obtained output and the correct answer becomes smaller. The value in the model updated here is a value such as a weight to be applied in connection of each unit of the neural network or a bias to be added. By repeating such training data sampling and model updating, the model is optimized so as to output a value closer to the correct answer. The model training is terminated based on, for example, the evaluation of the model with respect to the verification data set. The verification data set is a set of data formed of data that is not included in the training data set. Each time the training is repeated, the evaluation value of the model for the verification data is calculated, and the training is terminated when the evaluation value is not improved.

The input and output of the neural network are configured using a plurality of units so as to correspond to the input and output formats of the problem to learn. For example, when an image is input, the input to the model is a unit corresponding to each pixel of the image of height × width × channel. In addition, in the model that classifies the images, the output is a unit corresponding to each class to be classified, and the value is treated as the probability that the input is classified into the corresponding class. In the model that performs image segmentation, the output is a unit corresponding to the probability that each pixel of the image of height × width × class is classified into each class. For example, when determining the presence or absence of certain findings from the MCSLO image of the subject eye, the input to the model is the unit corresponding to each pixel of MCSLO image, the output is the unit corresponding to the probability of the presence of the findings to be detected, and the training data set is a set of pairs of the MCSLO image and the label indicating the presence or absence of the findings.

The analysis unit 2 reads the MCSLO image acquired by the image acquisition unit 1 from the storage unit 4 and inputs the image to the mathematical model. Then, the analysis unit 2 acquires the probability of each finding calculated according to the rules of the mathematical model. The analysis unit 2 stores the result of detection of the output findings in the storage unit 4.

### <MCSLO>

The MCSLO 200 is a device for obtaining a front image of the fundus by scanning a fundus with laser light including a plurality of wavelengths and receiving return light of the laser light from the fundus. The MCSLO 200 may be a device integrated with another ophthalmic device such as an optical coherence tomography (OCT) or a perimeter.

In the description below, the MCSLO 200 acquires the fundus image by two-dimensionally performing the scanning with the laser light to be focused on the spot on a observation surface based on the operation of the scanning unit.

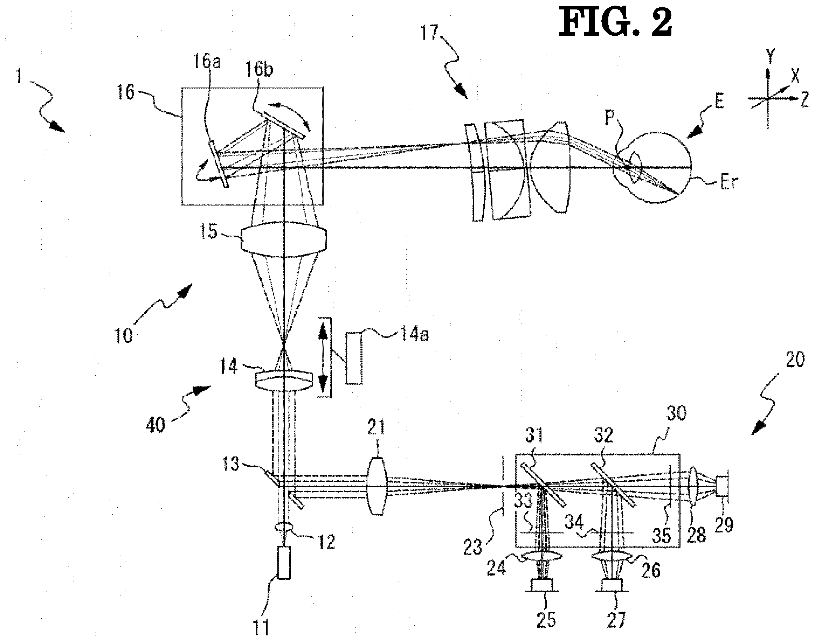

An optical system provided in the MCSLO 200 will be described with reference to FIG. 2. As illustrated in FIG. 2, the MCSLO 200 includes an irradiation optical system 10 and a light receiving optical system 20 (collectively referred to as an "imaging optical system"). The MCSLO 200 captures a fundus image with using these optical systems 10 and 20.

The irradiation optical system 10 includes at least a scanning unit 16 and an objective lens system 17. In addition, as illustrated in FIG. 2, the irradiation optical system 10 may further include a laser light emitting unit 11, a collimating lens 12, a perforated mirror 13, a lens 14 (a part of a diopter adjustment unit 40 in the present example), and a lens 15.

The laser light emitting unit 11 is a light source of the irradiation optical system 10. In the present example, the laser light from the laser light emitting unit 11 is used as illumination light emitted from the irradiation optical system 10 to a fundus Er. The laser light emitting unit 11 may include, for example, a laser diode (LD) and a super luminescent diode (SLD). The description of the specific structure is omitted, however, the laser light emitting unit 11 emits the light having at least equal to or more than one wavelength ranges. In the present example, the light of a plurality of colors is emitted from the laser light emitting unit 11 simultaneously or selectively. For example, in the present example, the light of four colors in total including three colors in the visible range of blue, green and red and one color in the infrared range is emitted from the laser light emitting unit 11. The light of each color can be emitted simultaneously or alternately. The three colors of blue, green, and red in the visible range are used for a color imaging, for example. The "simultaneous" here does not need to be strictly simultaneous, and there may be a time lag in the emission timing of the light of each wavelength. The time lag may be, for example, within a range in which a shift between the images due to eye movement is allowed in a fundus image formed based on the light of each wavelength.

For example, the color imaging is performed by emitting three colors of blue, green, and red from the light source 11 substantially at the same time. In addition, any one of the three colors in the visible range may be used for the visible fluorescence imaging. For example, blue light may be used for FA imaging (fluorescein fluorescence contrast imaging) which is a kind of visible fluorescence imaging. In addition, for example, green light may be used for FAF imaging (Fundus Auto-Fluorescence). That is, the green light may be used as excitation light for a fluorescent substance (for example, lipofuscin) accumulated in the fundus. In addition, for example, the light in the infrared range may be used for infrared fluorescent imaging as well as infrared imaging using fundus reflected light in the infrared range. For example, an IA image (indocyanine green fluorescence contrast imaging) is known as the infrared fluorescent imaging. In this case, it is preferable that the infrared light emitted from the laser light source 11 is set in a wavelength ranges different from the fluorescence wavelength of indocyanine green used in IA imaging.

The laser light is guided to the fundus Er in the path of the light ray illustrated in FIG. 2. That is, the laser light from the laser light emitting unit 11 passes through the collimating lens 12, passes through an opening portion formed in the perforated mirror 13, passes through the lens 14 and the lens 15, and then, goes toward the scanning unit 16. The laser light reflected by the scanning unit 16 passes through the objective lens system 17, and then, the fundus Er of the subject eye E is irradiated with the light. As a result, the laser light is reflected and scattered by the fundus Er or excites a fluorescent substance existing in the fundus, and then, generates fluorescence from the fundus. Those light rays (that is, reflected and scattered light, fluorescence, and the like) are emitted from the pupil as the return light.

In the present example, the lens 14 illustrated in FIG. 2 is a part of the diopter adjustment unit 40. The diopter adjustment unit 40 is used for correcting (reducing) the error of the diopter of the subject eye E. For example, the lens 14 can be moved in the optical axis direction of the irradiation optical system 10 by a drive mechanism 14a. Depending on a position of the lens 14, the diopter of the irradiation optical system 10 and the light receiving optical system 20 changes. Therefore, the error of the diopter of the subject eye E is reduced by adjusting the position of the lens 14, and as a result, the focusing position of the laser light can be set on the observation region (for example, the surface of retina) of the fundus Er. As the diopter adjustment unit 40, an optical system different from that illustrated in FIG. 2 such as a Badal optical system may be applied.

The scanning unit 16 (also referred to as an "optical scanner") is a unit for scanning the fundus with the laser light emitted from the light source (laser light emitting unit 11). In the description below, it is assumed that the scanning unit 16 includes two optical scanners having different laser light scanning directions unless otherwise specified. That is, the optical scanner includes an optical scanner 16a for main scanning (for example, for scanning in the X direction) and an optical scanner 16b for sub scanning (for example, for scanning in the Y direction). In the description below, the optical scanner 16a for main scanning is a resonant scanner, and the optical scanner 16b for sub-scanning is a galvano mirror. However, another optical scanner may be applied to each of the optical scanners 16a and 16b. For example, for each of the optical scanners 16a and 16b, in addition to other reflection mirrors (the galvano mirror, polygon mirror, resonant scanner, MEMS, and the like), an acousto-optical element (AOM) that changes the traveling (deflection) direction of light, or the like may be applied.

The objective lens system 17 is an objective optical system of the SLO 200. The objective lens system 17 is used to guide the laser light scanned by the scanning unit 16 to the fundus Er. Therefore, the objective lens system 17 forms a turning point P at which the laser light passed through the scanning unit 16 is turned. The turning point P is formed on the optical axis L1 of the irradiation optical system 10 and at a position optically conjugate with the scanning unit 16 with respect to the objective lens system 17. In the present disclosure, the "conjugate" is not necessarily limited to a complete conjugate relationship, and includes "substantially conjugate". That is, a case where the fundus image is displaced from the complete conjugate position within the range permitted in relation to the purpose of use (for example, observation, analysis, or the like) of the fundus image is also included in "conjugation" in the present disclosure. However, the objective optical system of the MCSLO 200 is not limited to the lens system, and may be a mirror system, a combination of the lens system and the mirror system, or another optical system.

The laser light passing through the scanning unit 16 passes through the objective lens system 17 to pass through the turning point P, and then, the fundus Er is irradiated with the laser light. Therefore, the laser light passing through the objective lens system 17 is turned around the turning point P according to the operation of the scanning unit 16 moves. As a result, in the present example, the fundus Er is two-dimensionally scanned with the laser light. The laser light applied to the fundus Er is reflected at the focusing position (for example, the surface of retina). In addition, the laser light is scattered by the tissues before and after the focusing position. The reflected light and the scattered light are emitted from the pupil as parallel light.

Next, the light receiving optical system 20 will be described. The light receiving optical system 20 includes one or a plurality of light receiving elements. For example, as illustrated in FIG. 2, a plurality of light receiving elements 25, 27, and 29 are included. In this case, the light from the fundus Er irradiated with the laser light emitted by the irradiation optical system 10 is received by the light receiving elements 25, 27, and 29.

As illustrated in FIG. 2, the light receiving optical system 20 in the present example may share the respective members arranged from the objective lens system 17 to the perforated mirror 13 with the irradiation optical system 10. In this case, the light from the fundus is guided to the perforated mirror 13 by retracing the optical path of the irradiation optical system 10. The perforated mirror 13 guides the light from the fundus Er to an independent optical path of the light receiving optical system 20 while removing at least a part of noise light due to the reflection from a cornea of the subject eye and the optical system inside the device (for example, the lens surface of the objective lens system).

The optical path branching member for branching the irradiation optical system 10 and the light receiving optical system 20 is not limited to the perforated mirror 13, and other beam splitters may be used.

The light receiving optical system 20 in the present example includes a lens 21, a pin hole plate 23, and a light separation unit (light separation unit) 30 on the reflected light path of the perforated mirror 13. In addition, lenses 24, 26, and 28 are provided between the light separation unit 30 and each of the light receiving elements 25, 27, and 29. In the present example, the light separation unit (light separation unit) 30 is used as a spectroscopic unit.

The pin hole plate 23 is arranged on the fundus conjugate plane and functions as a confocal diaphragm in the MCSLO 200. That is, when the diopter is appropriately corrected by the diopter adjustment unit 40, the light from the fundus Er that has passed through the lens 21 is focused at the opening of the pin hole plate 23. The pin hole plate 23 removes the light from positions other than the focal point (or focal plane) of the fundus Er, and the rest (light from the focal point) is mainly guided to the light receiving elements 25, 27, and 29.

The light separation unit 30 separates the light from the fundus Er. In the present example, the light from the fundus Er is wavelength-selectively separated by the light separation unit 30. In addition, the light separation unit 30 may also serve as an optical branching unit that branches the optical path of the light receiving optical system 20. For example, as illustrated in FIG. 2, the light separation unit 30 may include two dichroic mirrors (dichroic filters) 31 and 32 having different light separation characteristics (wavelength separation characteristics). The optical path in the light receiving optical system 20 is branched into three by the two dichroic mirrors 31 and 32. In addition, one of the light receiving elements 25, 27, and 29 is arranged at the end of each branched optical path.

For example, the light separation unit 30 separates the wavelength of the light from the fundus Er, and causes the three light receiving elements 25, 27, and 29 to receive the light having the different wavelength ranges. For example, the light of three colors of blue, green and red may be received by the light receiving elements 25, 27 and 29 one by one. In this case, a color image can be obtained from the results of light receiving by the light receiving elements 25, 27, and 29.

In addition, the light separation unit 30 causes the light in the infrared range used in the infrared image to be received by at least one of the light receiving elements 25, 27, and 29. In this case, for example, the fluorescence used in the fluorescence image and the infrared light used in the infrared image may be received by different light receiving elements.

The wavelength bands to which the respective light receiving elements 25, 27, 29 are sensitive may be different from each other. In addition, at least two of the light receiving elements 25, 27, and 29 may be sensitive to the wavelength ranges. Each of the light receiving elements 25, 27, and 29 outputs a signal (hereinafter, referred to as a received light signal) corresponding to the intensity of the received light. In the present embodiment, the received light signal is separately processed for each of the light receiving elements, and the image is generated. That is, in the present embodiment, up to three types of fundus images are generated in parallel.

### <Control Operation>

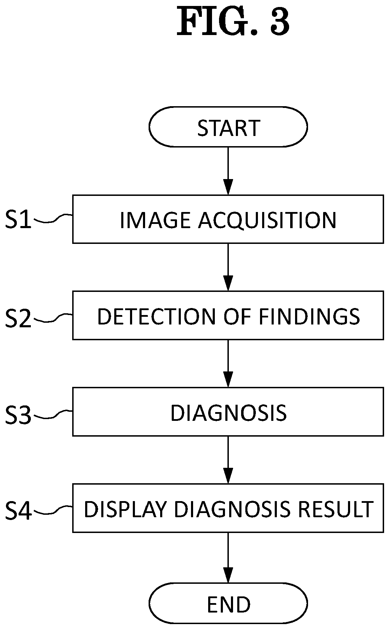

A control operation when the fundus image processing device 100 in the present example performs the image processing will be described based on FIG. 3. In an example below, a case will be described where the subject eye is automatically diagnosed by analyzing the MCSLO image captured by the MCSLO 200.

### (Step S1: Image Acquisition)

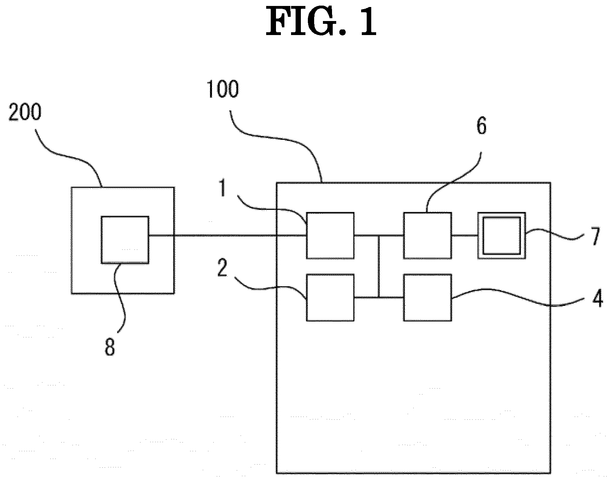

First, the image acquisition unit 1 acquires an MCSLO image. The image acquisition unit 1 acquires an image captured by the MCSLO 200 via, for example, wireless or wired communication means. The MCSLO image captured by the MCSLO is stored in the storage unit 8 (refer to FIG. 1) of the MCSLO 200. The image acquisition unit 1 receives the MCSLO image stored in the storage unit 8 from the MCSLO 200 and stores the image in the storage unit 4. The image acquisition unit 1 may directly acquire the image from the MCSLO 200 or may acquire the MCSLO image stored in an external storage device such as a USB memory.

### (Step S2: Detection of Findings)

Subsequently, the analysis unit 2 detects the findings by analyzing the acquired MCSLO image. For example, as described above, the analysis unit 2 analyzes the MCSLO image with using the mathematical model trained by the machine learning algorithm. For example, in the mathematical model, a combination of MCSLO image and a result of detection of findings is learned. The analysis unit 2 performs the detection of the findings by inputting the MCSLO image into the learning completed mathematical model.

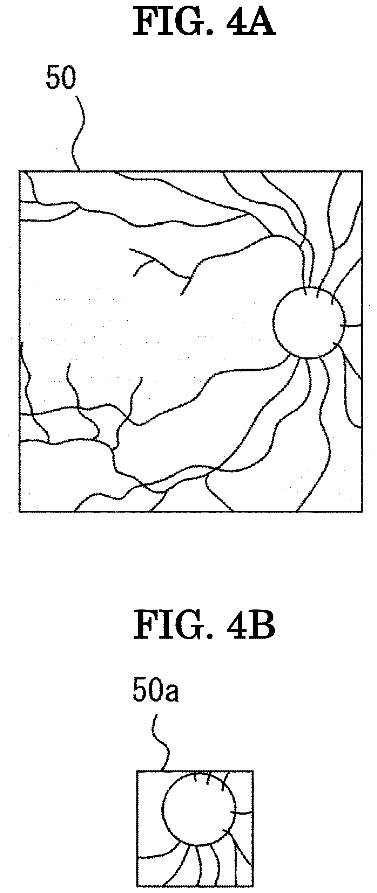

The mathematical model learns various combinations of images of the red channel, the green channel, and the blue channel. For example, the mathematical model may learn the image of each channel of red channel, green channel, blue channel, may learn a multi-channel image in which two channels of red channel and green channel, green channel and blue channel, or blue channel and red channel are combined, and may learn a multi-channel image in which3-channel of a red channel, a green channel, and a blue channel are combined. Of course, the mathematical model corresponding to each of these input formats may be caused to learn and may be used for the detection of findings. In addition, as illustrated in FIG. 4A and FIG. 4B, the mathematical model corresponding to each of the entire image 50 of the fundus image and the partial image 50a of the fundus image may be caused to appropriately learn. The analysis unit 2 outputs the findings by inputting the image of each channel, the image of a plurality of channels, the entire image 50 or the partial image 50a into the mathematical model according to the format of the image that the mathematical model learned. In addition, when a model that can use images of different sizes for learning and prediction such as used in segmentation is used as the mathematical model, the images of different sizes may be applied for learning and prediction. For example, the input at the time when the mathematical model learns may be used as a partial image and an area where the findings are present may be preferentially learned, or the input during the prediction may be used as the partial image and only the region of interest in the image may be processed by the mathematical model.

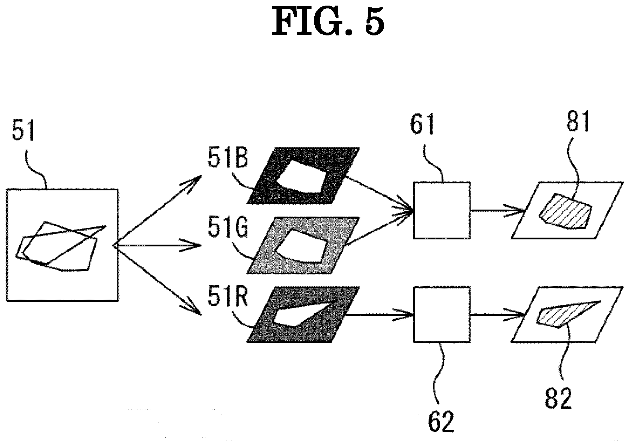

In the MCSLO image, the depth of image varies depending on the wavelength. Therefore, a plurality of mathematical models in which the wavelengths of the images to be learned are different from each other may be prepared. For example, as illustrated in FIG. 5, analysis unit 2 may detect the findings with using the surface layer mathematical model 61 that detects the findings of the retinal surface layer and the deep layer mathematical model 62 that detects the findings of the retinal deep layer. For example, in the surface layer mathematical model 61, the images of the green channel and the blue channel in which the tissue of the retinal surface layer is mainly imaged are learned, and in the deep layer mathematical model 62, a red channel image in which the tissue of the retinal deep layer is mainly imaged is learned. The analysis unit 2 inputs the blue channel image 51B and the green channel image 51G in the MCSLO image 51 captured from the subject eye into the surface layer mathematical model 61, and then, outputs the findings 81 of the retinal surface layer. Similarly, the analysis unit 2 inputs the red channel image 51R into the deep layer mathematical model 62, and the, outputs the findings 82 of the retinal deep layer. As described above, due to using the mathematical models different from other for each channel, even if the positions of the findings 81 of the retinal surface layer and the findings 82 of the retinal deep layer overlap as illustrated in FIG. 5, the findings 81 and the findings 82 can be detected separately.

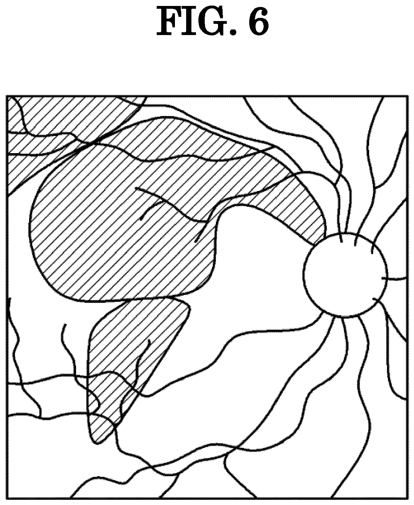

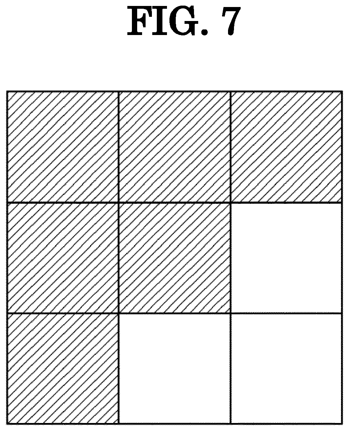

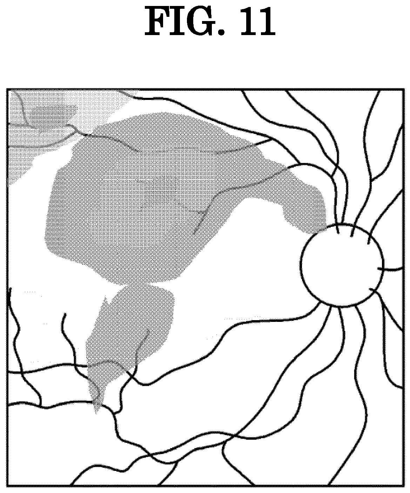

As a method of outputting the findings, for example, as illustrated in FIG. 6, a map image obtained by the segmentation of each finding with respect to the MCSLO image may be output. In addition, as illustrated in FIG. 7, a map image indicating the presence or absence of each finding in each divided area of the image may be output. In addition, as illustrated in FIG. 11, a map image (attention map) indicating an attention area of the mathematical model for identifying the presence or absence of each finding of the image may be output.

### (Step S3: Diagnosis)

The analysis unit 2 performs the diagnosis of the subject eye based on the result of detection of the findings obtained by the analysis of the MCSLO image. For example, the analysis unit 2 identifies the disease (diabetic retinopathy, macular anterior membrane, and the like) expected from the image based on the map image of the findings, and obtains the result of identification of each disease. The analysis unit 2 may identify a disease with using a mathematical model that learned the result of identification of the disease. For example, the analysis unit 2 may output the probability of each disease, or the like by inputting the map image of the findings into the mathematical model.

### (Step S4: Display Result of Diagnosis)

The display control unit 6 displays the result of diagnosis of each disease obtained in step S3 on the display unit 7. At this time, the display control unit 6 may display the map image of the findings obtained in step S2 as confirmation information for the result of diagnosis. As a result, the user can confirm not only the final result of diagnosis but also the basis of the diagnosis.

As described above, the ophthalmologic image processing device 100 automatically performs the detection of the findings from the MCSLO image. As a result, even a user unfamiliar with the multi-color fundus image can easily perform the image interpretation or the diagnosis. In addition, in the MCSLO image, it is possible to acquire the more information by detecting the findings from the images of each wavelength such as red, green, blue, or a multi-channel image which is a combination thereof.

As described in the present example, it is possible to detect the findings more accurately because the mathematical model learned by the machine learning algorithm is used. In addition, due to using a mathematical model learning images for different wavelengths, it is possible to efficiently perform the detection even when there is an overlap in the findings of each layer.

Even though tens of thousands of images are needed to generate one mathematical model, the MSCLO images, or the like are not yet widespread compared to the fundus images used in the related art, it is difficult to obtain a large number of images. In such cases, as described in the present example, by firstly applying the mathematical model for detecting the findings and by performing the diagnosis using the result, it is possible to construct a good mathematical model even by learning a relatively small data set.

The fundus image processing device 100 may perform only the outputting of the findings. In this case, the user may perform the diagnosis of the subject eye based on the findings output by the fundus image processing device 100. For example, the user confirms the findings output to the display unit 7 and inputs the result of diagnosis using the operation unit such as a touch panel. As described above, even when the user perform the diagnosis of the subject eye, since the findings according to the depth of the fundus can be confirmed, it is possible to obtain the information for performing the more accurate diagnosis.

In the example described above, the detection of the findings was performed with using the neural network, but not limited thereto. For example, other machine learning algorithms such as the random forest, boosting, and the SVM may be used.

The image acquisition unit 1 may acquire an image from a server or the like. For example, a plurality of measurement results captured by many kinds of devices are stored in the server via the network, and the image acquisition unit 1 may be able to acquire the image data captured by another MCSLO device from the server. The image acquisition unit 1 may also acquire an image from an electronic medical chart system that manages registration information and examination information of the subject patient.

Various types of images may be used in the image analysis in which the mathematical model is used. For example, in addition to the multi-color SLO image, a tomographic image, a fundus image, a blood vessel image, an anterior segment image, and the like may be input to the mathematical model. Those images are captured by various ophthalmologic imaging devices such as an OCT device, a fundus camera, an anterior segment observation camera, a slit lamp, and a scheimpflug camera.

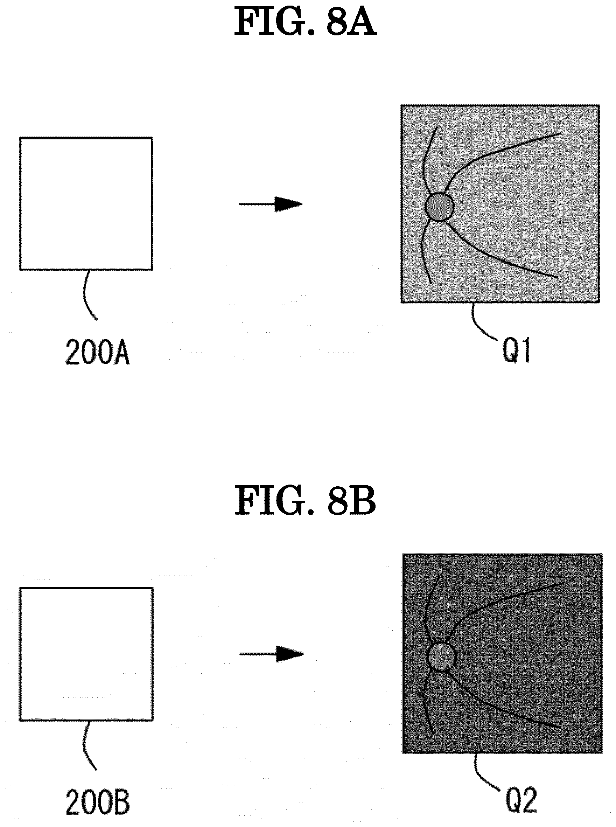

As illustrated in FIG. 8A and FIG. 8B, multi-color fundus images Q1 and Q2 captured by different imaging devices have different color balances. Therefore, for example, in the mathematical model that learns the fundus image captured by a first imaging device 200A, the image captured by a second imaging device 200B cannot be correctly identified.

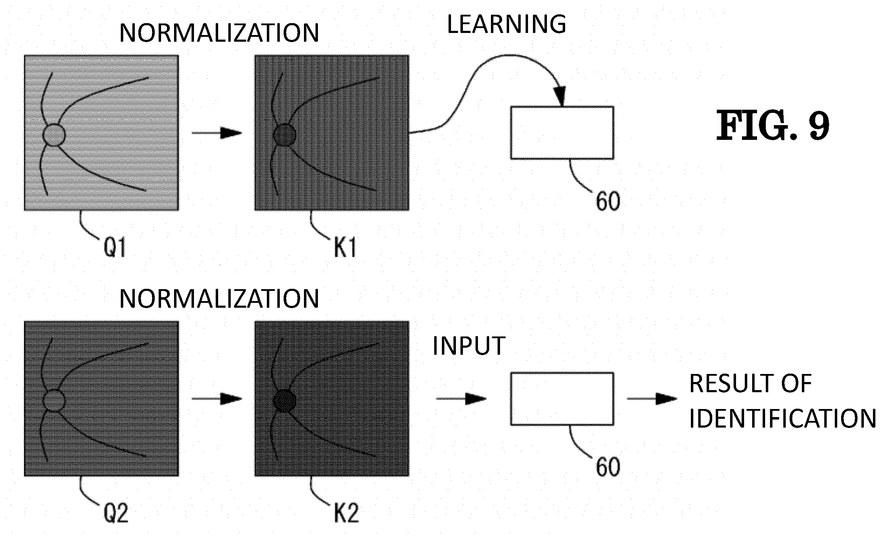

Therefore, the analysis unit 2 may normalize the luminance of each channel of the fundus image before inputting the images into the mathematical model so that the fundus images captured by different imaging devices can be identified in the same mathematical model. When the images are normalized, even if the imaging devices are different, the images will be similar as long as they are captured at the similar wavelengths. For example, as illustrated in FIG. 9, a fundus image K1 obtained by normalizing the fundus image Q1 captured by the first imaging device 200A and a fundus image K2 obtained by normalizing the fundus image Q2 captured by the second imaging device 200B are the images look like the same. For this reason, a mathematical model 60 that learned the fundus image K1 obtained by normalizing the fundus image Q1 can identify the fundus image K2 obtained by normalizing the fundus image Q2. That is, if the image is normalized before input into the mathematical model, even if fundus image is captured by an imaging device different from the imaging device used for learning, the fundus image can be identified by the same mathematical model. As a method of normalization, for example, for each channel of the fundus image, processing of subtracting the average of the channel and dividing the result by the standard deviation of the channel can be considered. Of course, other normalization methods may be used. As the normalization, the image resolution may be made uniform in addition to the luminance.

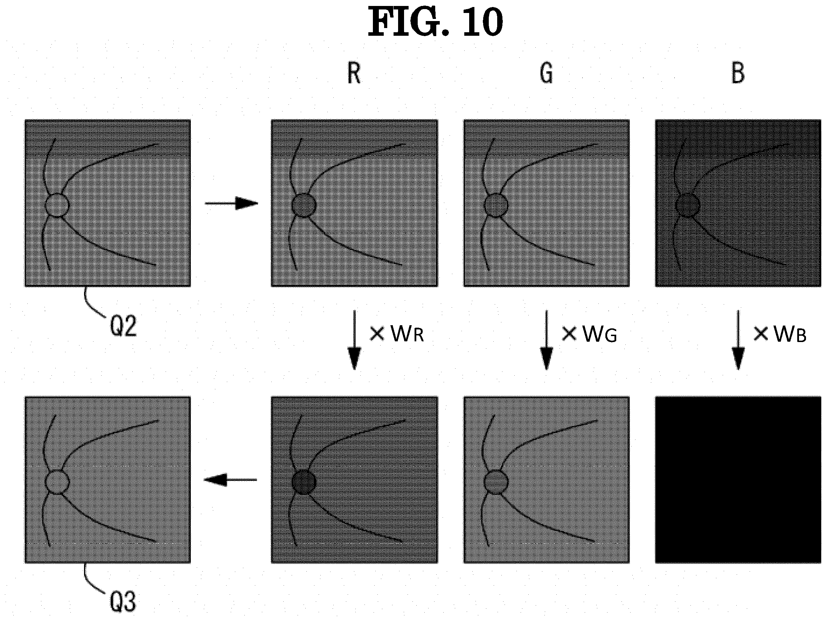

When inputting the fundus image captured by the imaging device different from the imaging device used for the mathematical model to learn, the analysis unit 2 may input to the mathematical model after weighting the brightness of the fundus image of each channel appropriately. For example, as illustrated in FIG. 10, by weighting each channel of the fundus image Q2 captured by the second imaging device 200B by the weights wR, wG, and wB, respectively, the above fundus image Q2 is converted into a fundus image Q3 similar to the fundus image Q1 captured by the first imaging device 200A. As described above, by weighting the images so that the fundus image (second fundus image) input to the mathematical model is close to the specification of the learning completed fundus image (first fundus image), even the images captured by the different imaging devices can be identified by one mathematical model. As methods of calculating the weight, the weight may be calculated with using, for example, the average or variance of the luminance of representative image of each imaging device, or the user may manually set an appropriate weight. In addition, if the weights used when the imaging device generates the images for each channel is known, that weight may be used for the calculation. In addition to the weighting, another correction value such as adding an offset value may be used.

Even in a monochromatic fundus image captured by the different imaging devices, since the magnitude of the luminance is different, the normalization of the brightness or weighting processing may be performed.

In addition, for the normalization or weighting processing, a fundus imaging device (for example, a fundus camera or a scanning fundus imaging device) that includes a white light source and receives the fundus reflected light from the white light source, and images a color fundus image, can be applied. In this case, the luminance of each pixel of the color fundus image is acquired as the brightness value for each RGB. Therefore, with respect to the luminance value for each RGB, the normalization or the weighting processing may be performed between the first color fundus image captured by the first imaging device and the second color fundus image captured by the second imaging device.

### Reference Signs List

- 100: image processing device
- 1: image acquisition unit
- 2: analysis unit
- 4: storage unit
- 6: display control unit
- 7: display unit
- 8: storage unit
- 200: multi-color SLO

## Claims

1. A fundus image processing device that processes a fundus image of a subject eye, comprising:
image acquisition means for acquiring the fundus image captured by imaging means; and
analysis means for analyzing the fundus image,
wherein the imaging means performs scanning with measurement light including a plurality of wavelengths by scanning means, and generates the fundus image based on a received light signal for each wavelength obtained from a light receiving element which receives the measurement light reflected from a fundus of the subject eye, and
the analysis means detects findings for the fundus image generated based on the received light signal for each wavelength.

2. The fundus image processing device according to claim 2,
wherein the measurement light includes at least one of red light, green light, and blue light.

3. The fundus image processing device according to claim 1 or 2,
wherein the analysis means detects findings for each of the fundus images generated for each wavelength.

4. The fundus image processing device according to any one of claims 1 to 3,
wherein the analysis means detects findings for a multi-channel image in which two or more of the fundus images generated for each wavelength are combined.

5. The fundus image processing device according to any one of claims 1 to 4,
wherein the analysis means analyzes the fundus image with using a mathematical model trained by a machine learning algorithm.

6. The fundus image processing device according to claim 5,
wherein the analysis means analyzes the fundus image with using a mathematical model which is different for each wavelength corresponding to an image.

7. The fundus image processing device according to claim 6,
wherein the analysis means analyzes the fundus image with using a surface layer mathematical model which learned fundus images of a first channel and a deep layer mathematical model which learned fundus images of a second channel different from the first channel.

8. The fundus image processing device according to any one of claims 1 to 7,
wherein the imaging means is a scanning laser ophthalmoscope.

9. The fundus image processing device according to any one of claims 1 to 8,
wherein the analysis means outputs reference information used for diagnosis of the subject eye based on a detection result of findings for the fundus image.

10. The fundus image processing device according to claim 5,
wherein the analysis means normalizes the fundus image before inputting the fundus image into the mathematical model.

11. The fundus image processing device according to claim 5,
wherein in a case where the analysis means inputs a second fundus image, which is captured by second imaging means different from first imaging means capturing a first fundus image used for learning the mathematical model, into the mathematical model, the analysis means weights each channel of the second fundus image.

12. A fundus image processing program executed in a fundus image processing device that processes a fundus image of a subject eye, and executed by a processor of the fundus image processing device to cause the fundus image processing device to perform:
a generation step of performing scanning with measurement light including a plurality of wavelengths by scanning means, and generating the fundus image based on a received light signal for each wavelength obtained from a light receiving element which receives the measurement light reflected from a fundus of the subject eye;
an image acquisition step of acquiring the fundus image generated in the generation step; and
an analysis step of detecting findings for the fundus image generated based on the received light signal for each wavelength.
